# EUROPEAN PATENT APPLICATION

(11) **EP 0 713 705 A1**
(43) Date of publication of application: **29.05.1996**
(21) Application number: 95913331.5
(22) Date of filing: 24.03.1995
(51) Int. Cl.: A61K 31/70, C07H 21/04

(54) **NOVEL ANTI-HIV DRUG**

(30) Priority: 25.03.1994 JP 92809/94; 25.03.1994 JP 92810/94
(71) Applicant: KAJI, Akira, Tokyo 203 (JP)
(72) Inventor: KAJI, Akira, Tokyo 203 (JP)
(74) Representative: Flaccus, Rolf-Dieter, Dr.
(86) International application number: JP9500543
(87) International publication number: WO9526190

(57) **Abstract**

An anti-HIV drug containing as the active ingredient one or more phosphodiester-linkage oligodeoxynucleotides represented by the following general formula (I): Pn(Gp)mG (I) wherein G represents a 2'-deoxyguanosine structure; p represents a phosphate residue forming an ester linkage with the adjacent G, and the terminal p forms an ester linkage with the 5'-hydroxyl group of the 5'-terminal G, while each of the p's positioned between two G's forms ester linkages with, respectively, the 3'-hydroxyl group of the lefthand G and the 5'-hydroxyl group of the righthand G in the formula; n represents a number of 0 or 1; and m represents a number of 4 to 15, provided m is a number of 6 to 15 when n is 0.

## Description

### FIELD OF THE INVENTION

The present invention relates to an anti-HIV(Human Immunodeficiency Virus) agent containing as an active ingredient a phosphodiester bonding type oligodeoxyguanylic acid which shows an excellent anti-viral action for diseases caused by HIV.

### BACKGROUND ART

In the below description, C is 2'-deoxycytidine and A is 2'-deoxyadenosine and T is 2'-deoxythymidine respectively.

As a previous finding on phosphodiester bonding type homooligomers of DNA, Agrawal et al reported that the 15-bases of A, G, C or T were active for inhibition of HIV replication, and the effect was due to the length of the 15-bases and was independent of the kind of the base. And as for the effectiveness, it was reported that due to the lengthy homooligomers they might favorably act to reverse transcriptase as a template and be competitive to HIV nucleic acid. (Proc. Nat. Acad. Sci. USA, 85, 7079, 1988)

Majumdar et al. reported that the 28-base of the phosphodiester type bonding oligomer of C was active for inhibition of HIV replication (Biochemistry, 28, 1340, 1989).

By summing up these Crooke described that the activity of homopolymers was in the order that C was most active and between T and A, T was a little bit stronger than A and by showing the 28-base of C as an example its activity was length dependent (Anti Cancer Drug Design, 6, 609, 1991).

### DISCLOSURE OF THE INVENTION

The inventor investigated the anti-viral effect of phosphodieser bonding type oligodeoxynucleotides.

The inventor made an extensive research on an anti-HIV activity of homooligomers of A, G, C and T in which the chain length was shortened. As the result, in G not only the 15-base but the 4-base showed the anti-HIV activity, however, the 15-bases of A, C or T did not show any activity. Further the inventor got a new finding that the 5'-phosphrylation and the 3'-phosphrylation increased the activity.

Accordingly, the invention provides an anti-HIV agent which comprises as an active ingredient one or more of phosphodiester bonding type oligodeoxynucleotides of the below formula (I)

pₙ(Gp)ₘG (I)

wherein G represents 2'-deoxyguanosine structure, p is a phosphoric acid residue which forms ester bonding with the neighboring G, the terminal p forms a monoester with 5' hydroxyl of 5'-terminal G, the each in-between p of two Gs forms ester bondings with 3' hydroxyl of the left side G and with 5' hydroxyl of the right side G respectively, n is 0 or 1, m is an integer of 4∼15. But in the case n is 0, m is an integer of 6∼15.

Detailed explanation of the invention is mentioned below.

Phosphodiester bonding type oligodeoxyguanylic acids which are active ingredients of the anti-HIV agents of the invention can be synthesized by the solid phase phosphoramidite method used in oligodeoxynucleotide synthesis, or by the solid or the liquid phase triester method, etc., as usually used. Examples are as follows.

A support binding a commercial and functionally protected. nucleic acid monomer is used as a starting material which is subjected to bind sequentially as needed to deoxynucleic acid monomer whose amino and acidic hydroxyl groups are protected to afford a crude oligodeoxynucleotide which is purified after the deprotection. For the synthesis of oligodeoxynucleotides it is favorable to select a method among the phosphoramidite and the triester methods, etc.

In a case of the solid phase synthesis by the phosphoramidite method, a starting material is a compound in which 3'-terminal nucleoside of an aimed DNA attaches to a commercial controlled-pore glass (CPG). After 4,4'-dimethoxytrityl group, the protecting group of 5'-hydroxyl in this nucleoside, is deprotected by trifluoroacetic acid, etc., the amidite reagent (5'-O-dimethoxytrityl-3'-methoxy-N, N-diisopropylaminophosphinedeoxynucleoside) is subjected to the condensation by tetrazole whose amino group in the nucleic acid base as a commercial monomer corresponding to the second from 3'-terminal side is protected by benzoyl or isobutylyl group. The acetylation of the unreacted 5'-hydroxyl group by acetic anhydride and dimethylaminopyridine, etc., to avoid a byproduct production is followed by the oxidation of the phosphinic acid part with iodine to lead to a triester derivative. The above reaction is repeated until the aimed chain length is obtained. In a case of synthesis of a compound of the general formula pₙ(Gp)ₘG in which n equals 1, the condensation to 5'-terminal with phosphorylation reagent (2-[2-(4,4'-dimethoxytrityloxy)]ethylsulfonyl-(2-cyanoethyl)-(N,N-diisopropyl)phosphoramidite) is followed by the oxidation of the phosphinic acid part with iodine to lead to a triester derivative which is then treated with thiophenol to convert each internucleotide from the triester to the diester. The diester is treated with ammonium hydroxide to detach from the support and to deblock simultaneously the base part to afford a crude oligodeoxynucleotide which is purified by ionexchange HPLC, gel electrophoresis, etc., to give the aimed oligodeoxynucleotide.

The structure of the oligodeoxynucleotide synthesized as above can be confirmed by DNA sequence analysis.

The phosphodiester bonding type oligodeoxyguanylic acids which are the active ingredients of the anti-HIV agents of the invention can also be bought commercially.

### Experimental examples

Experimental examples are given below to explain the invention in more details, but the invention is not limited thereby in any way.

### 〈Anti-AIDS virus (HIV) activity tests using MT-4 cell (T cell system)〉

To a 96-well microplate which contained the phosphodiester bonding type oligodeoxynucleotide diluted stepwise were placed MT-4 cells (6x10⁴ cells per well). The cells were then infected with AIDS virus [HIV-1(III B) : ca. 50TCID₅₀ (50% tissue culture infectious doses)/well] and were incubated in a 5% CO₂ atmosphere at 37°C for 5 days. For evaluation of the inhibition of the cytopathic effect by the infection, the number of viable cells was counted after 5 days' incubation. The results are shown in Table 1.

In the formula of each oligodeoxynucleotide in the table, the meaning of each symbol is identical to the definition described in the above formula (I).

**Table 1**

| No. | | ED₅₀ | CD₅₀ | SI |
|---|---|---|---|---|
| 1 | GpGpGpGpG | not effective at 100µg/ml | ≧100µg/ml | |
| 2 | GpGpGpGpGpG | 49.2µg/ml | ≧100µg/ml | ≧2.0 |
| 3 | GpGpGpGpGpGpGpGpG | 4.4µg/ml | ≧100µg/ml | ≧22.7 |
| 4 | GpGpGpGpGpGpGpGpGpGpGpG | 4.0µg/ml | ≧100µg/ml | ≧25.0 |
| 5 | GpGpGpGpGpGpGpGpGpGpGpGpGpGpG | 5.8µg/ml | ≧100µg/ml | ≧17.2 |
| 6 | pGpGpG | not effective at 100µg/ml | ≧100µg/ml | |
| 7 | pGpGpGpG | 17.1µg/ml | ≧100µg/ml | ≧5.8 |
| 8 | GpGpGpGp | 20.4µg/ml | ≧100µg/ml | ≧4.9 |
| 9 | pGpGpGpGpGpG | 5.9µg/ml | ≧100µg/ml | ≧16.9 |
| 10 | pGpGpGpGpGpGpGpG | 5.8µg/ml | ≧100µg/ml | ≧17.2 |
| 11 | pGpGpGpGpGpGpGpGpGpG | 2.3µg/ml | ≧100µg/ml | ≧43.5 |
| 12 | pApApApApApApApApApA | not effective at 100µg/ml | ≧100µg/ml | |
| 13 | pCpCpCpCpCpCpCpCpCpC | not effective at 100µg/ml | ≧100µg/ml | |
| 14 | pTpTpTpTpTpTpTpTpTpT | not effective at 100µg/ml | ≧100µg/ml | |

The phosphodiester bonding type oligodeoxyguanylic acids of the formula (I), in which n is zero as the active ingredients of the anti-HIV agents of the invention, showed effect from the 6-base. The 9-base showed the excellent anti-HIV viral activity. Further the phosphodiester bonding type oligodeoxyguanylic acids of the formula (I), in which n is one showed effect from the 4-base (Table 1, No. 7). The 6-base showed the excellent anti-HIV viral activity (Table 1, No. 9). In adding no toxicity was shown at the concentration of 100µg/ml. On the other hand, in A, C and T no activity was recognized even in the 10-bases, however, it was recognized that the efficacy of the phosphodiester bonding type oligodeoxyguanylic acids was especially remarkable.

Further, the 9-base of the phosphodiester bonding type oligodeoxyguanylic acid was administered to mice intravenously at the dose of 20mg/Kg or orally at the dose of 100mg/Kg and no death was observed.

Next, in the compounds of the formula (Gp)ₘG (m=5∼11) and in the compounds of the formula p(Gp)ₘG (m=5∼9) the administration dose and the preparations are explained.

Each phosphodiester bonding type oligodeoxyguanylic acid described above can be administered by itself or with a usual vehicle to animals and humans. As for an administration form there is no special restriction and a proper form is selected and used as required. Illustrative of the oral administration are tablets, capsules, granules, fine granules, powders, etc., and those of the parenteral administration are injections, suppositories, etc.

In order to exert an anticipated efficacy as oral preparations the dose for each patient depends on age, body weight and the severity of the disease, though, the daily dose for a usual adult is between 0.1 and 6 g which are favorably administered in several times.

In the invention, the oral preparations such as tablets, capsules, granules, etc., can be prepared by the conventional method using starch, lactose, sugar, mannite, carboxymethycellulose or inorganic salts.

In this kind of preparations, except to the above excipients other additives can be properly used such as binders, disintegraters, surfactants, lubricants, flowing promoters, sweeteners, colorings, flavors, etc. Each example is as shown below.

### [Binders]

Starch, dextrin, Arabic gum powder, gelatin, hydroxypropylstarch, methylcellulose, sodium carboxymethylcellulose, hydroxypropylcellulose, crystalline cellulose, ethylcellulose, polyvinylcellulose, macrogol.

### [Disintegraters]

Starch, hydroxypropylstarch, sodium carboxymethylcellulose, calcium carboxymethylcellulose, carboxymethylcellulose, low substituted hydroxypropylcellulose.

### [Surfactants]

Sodium laurylsulfate, soya lecithin, sucrose fatty acid ester, polysorvate 80.

### [Lubricants]

Talc, wax, hydrogenated vegetable oil, sucrose fatty acid ester, magnesium stearate, polyethylene glycol.

### [Flowing promoters]

Light anhydrous silicic acid, dry aluminum hydroxide gel, synthetic aluminum silicate, magnesium silicate.

The above phosphodiester bonding type oligodeoxynucleotides can be administered as suspensions, liquid emulsions, syrups or elixirs. For these dosage forms, flavorings or colorings can be included.

In order to exert an anticipated efficacy as parenteral preparations the dose for each patient depends on age, body weight and the severity of the disease, though, the daily dose of the above phosphodiester bonding type oligodeoxynucleotides for adults is usually between 1∼100mg which are administered by intravenous injection, i.v. drip infusion, subcutaneous injection or intramuscular injection.

The parenteral preparations can be prepared by the conventional method. Water for injection, physiological saline solution, aqueous glucose solution, vegetable oil for injection, sesame oil, peanut oil, soybean oil, corn oil, propylene glycol, polyethylene glycol or etc., can generally be used as diluents.

The formulations can contain antiseptics, preservatives or stabilizers as required. From the view point of stability, the parenteral preparations are filled into vials or etc., and frozen and dried by the conventional lyophilizing technology. Liquid preparations can be prepared again just before use by using the lyophlisastes. Further there may be appropriately added isotonic agents, stabilizers, preservatives, soothing agents or etc., as required.

As the other parenteral preparations, externally applicable preparations such as liquids, ointments, etc., and suppositories for rectal administration can be illustrated and all these can be prepared by conventional methods respectively. Working examples

Examples are given below to explain the invention in more details, but the invention is not limited thereby in any way.

### Example 1

| | |
|---|---|
| ① Crystalline cellulose | 84.5g |
| ② Magnesium stearate | 0.5g |
| ③ Calcium carboxymethylcellulose | 5g |
| ④ GpGpGpGpGpGpGpGpG | 10g |
| Total | 100g |

According to the above formulation ①, ④ and a part of ② were mixed uniformly. The mixture was compressed and then pulverized to powder which was mixed with ③ and the remaining ②. The mixture was compressed to form tablets (200mg/tablet) by a tablet machine.

In this one tablet, 20mg of GpGpGpGpGpGpGpGpG is included and for adults 4∼8 tablets per day are administered in several times.

### Example 2

| | |
|---|---|
| ① Crystalline cellulose | 34.5g |
| ② 10% ethanolic hydroxypropylcellulose solution | 50g |
| ③ Calcium carboxymethylcellulose | 5g |
| ④ Magnesium stearate | 0.5g |
| ⑤ GpGpGpGpGpGpGpGpG | 10g |
| Total | 100g |

According to the above formulation ①, ② and ⑤ were mixed to a uniform blend. The blend was kneaded by a conventional method and granulated by a pressing granulation machine, and after drying and pulverization the powder was mixed with ③ and ④. The mixture was compressed to form tablets (200mg/tablet) by a tablet machine.

In this one tablet, 20mg of GpGpGpGpGpGpGpGpG is included and for adults 4∼8 tablets per day are administered in several times.

### Example 3

| | |
|---|---|
| ① Crystalline cellulose | 84.5g |
| ② Magnesium stearate | 0.5g |
| ③ Calcium carboxymethylcellulose | 5g |
| ④ pGpGpGpGpGpG | 10g |
| Total | 100g |

According to the above formulation ①, ④ and a part of ② were mixed uniformly. The mixture was compressed and then pulverized to powder which was mixed with ③ and the remaining ②. The mixture was compressed to form tablets (200mg/tablet) by a tablet machine.

In this one tablet, 20mg of pGpGpGpGpGpG is included and for adults 4∼8 tablets per day are administered in several times.

### Example 4

| | |
|---|---|
| ① Crystalline cellulose | 34.5g |
| ② 10% ethanolic hydroxypropylcellulose solution | 50g |
| ③ Calcium carboxymethylcellulose | 5g |
| ④ Magnesium stearate | 0.5g |
| ⑤ pGpGpGpGpGpG | 10g |
| Total | 100g |

According to the above formulation ①, ② and ⑤ were mixed to a uniform blend. The blend was granulated by a pushing granule machine in a conventional way, and after drying and pulverization the powder was mixed with ③ and ④. The mixture was compressed to form tablets (200mg/tablet) by a tablet machine.

In this one tablet, 20mg of pGpGpGpGpGpG is included and for adults 4∼8 tablets per day are administered in several times.

### Example 5

| | |
|---|---|
| ① Distilled water for injection | appropriate |
| ② Glucose | 200mg |
| ③ GpGpGpGpGpGpGpGpG | 10mg |
| Total | 15ml |

In the distilled water for injection ② and ③ were dissolved. The solution was filled into ampoules which were sterilized at 121°C for 15 min. to afford injections.

### Example 6

| | |
|---|---|
| ① Distilled water for injection | appropriate |
| ② Glucose | 200mg |
| ③ pGpGpGpGpGpG | 10mg |
| Total | 15ml |

In the distilled water for injection ② and ③ were dissolved. The solution was filled into ampoules which were sterilized at 121°C for 15 min. to afford injections.

### EFFECT OF THE INVENTION

The novel anti-HIV agents of the invention are very effective for the treatment of the HIV (AIDS virus) induced diseases such as AIDS (Acquired Immune Deficiency Syndromes) and ARC (AIDS Related Complex).

## Claims

1. An anti-HIV agent which comprises as an active ingredient one or more of phosphodiester bonding type oligodeoxynucleotides of the below formula (I)
pₙ(Gp)ₘG (I)
wherein G represents 2'-deoxyguanosine structure, p is a phosphoric acid residue which forms ester bonding with the neighboring G, the terminal p forms a monoester with 5'-hydroxyl of 5'-terminal G, the each in-between p of two Gs forms ester bondings with 3'-hydroxyl of the left side G and with 5'-hydroxyl of the right side G respectively, n is 0 or 1, m is an integer of 4∼15. But in the case n is 0, m is an integer of 6∼15.
